# EUROPEAN PATENT SPECIFICATION

(11) **EP 4 360 538 B1**
(45) Date of publication and mention of the grant of the patent: **30.04.2025**
(21) Application number: 23163481.7
(22) Date of filing: 22.03.2023
(51) Int. Cl.: A61B 5/00, A61B 5/145

(54) **APPARATUS AND METHOD FOR ESTIMATING CONCENTRATION OF ANALYTE COMPONENT**
VORRICHTUNG UND VERFAHREN ZUR SCHÄTZUNG DER KONZENTRATION EINER ANALYTKOMPONENTE
APPAREIL ET PROCÉDÉ D'ESTIMATION DE LA CONCENTRATION D'UN CONSTITUANT D'ANALYTE

(30) Priority: 27.10.2022 KR 20220140161
(43) Date of publication of application: 01.05.2024
(73) Proprietor: Samsung Electronics Co., Ltd., Suwon-si, Gyeonggi-do 16677 (KR)
(72) Inventor: Eom, Kun Sun, Suwon-si, Gyeonggi-do (KR); Park, Jin Young, Suwon-si, Gyeonggi-do (KR); Jung, Myoung Hoon, Suwon-si, Gyeonggi-do (KR); Kim, Yoon Jae, Suwon-si, Gyeonggi-do (KR); Moon, Hyun Seok, Suwon-si, Gyeonggi-do (KR); Hwang, Jeong Eun, Suwon-si, Gyeonggi-do (KR)
(74) Representative: Grünecker Patent- und Rechtsanwälte PartG mbB

(56) References cited:
- WO-A1-2021/156117
- KR-A- 20210 101 896
- US-A1- 2016 302 706
- US-A1- 2019 192 057
- US-A1- 2022 167 883
- US-A1- 2023 047 540

## Description

### BACKGROUND

### 1. Field

The present disclosure relates to an apparatus and method for estimating the concentration of an analyte component, and more particularly to a technology for dynamically setting a sensor operating condition based on a change in skin.

### 2. Description of the Related Art

Reactive oxygen species act as an important biological defense factor such as white blood cells protecting the body against infections. However, it has been known that excessive generation of reactive oxygen species in the body may lead to various tissue diseases. Common factors that cause the reactive oxygen species include stress, alcohol, peroxides, medicine, and the like. The reactive oxygen species produced by these factors may cause cranial nerve diseases, circulatory diseases, cancer, digestive tract diseases, liver diseases, arteriosclerosis, renal diseases, diabetes, aging, and the like. Human bodies have a series of antioxidant defense systems to protect against oxygen toxicity. For normal operation of the systems, it is essential to consume sufficient antioxidants such as vitamin E, vitamin C, carotenoid, flavonoid, etc., and it is important to eat as many foods that are rich in antioxidants as possible for an effective antioxidant action. Accordingly, there is a need for an apparatus for easily identifying the amount of antioxidant components in the body.
KR 2021 0101896 A relates to a PPG (photoplethysmography) signal measurement system. Specifically, it detects the human skin color and adjusts the intensity of the LED based on this to reduce errors due to race and skin color to improve the accuracy of the pulsation cycle signal of PPG measured in blood vessels. The purpose of KR 2021 0101896 A is to provide a device and a method for improving the quality of the PPG pulsation cycle signal using a skin color detector in a blood glucose meter that can improve the accuracy of the cycle signal. The device for improving the quality of the PPG pulsation cycle signal using the skin color detector in the blood glucose meter according to KR 2021 0101896 A includes a white LED driver that operates a white light LED to irradiate the skin of the measurement subject before PPG measurement, a skin reflected light detection unit that measures the light irradiated and reflected on the skin of the measurement subject, a skin color estimation unit that converts the light measured to be reflected from the skin into a color code and determines the skin color type based on the color code, a LED driving current setting unit that sets the intensity of the current applied to the LED for PPG measurement according to the determined skin color type, and a PPG signal measuring unit that receives the set current and measures the PPG signal.
WO 2021/156117 A1 discloses that the device for detecting health-related values of a user can have a sensor for detecting the skin color of a user, e.g. a spectral sensor that is suitable for determining the skin color of the user. This information can then be used to adapt the intensity and spectral emission of a light source for further recording of health-related values of a user by the multispectral camera.
US 2016/302706 A1 mentions that ambient light and skin color make it difficult to extract a user's heart rate from a PPG signal. In US 2016/302706 A1, it is thus described that the effect of skin color can be reduced by changing the intensity of the PPG light source, the wavelength of the light emitted from the light source, and/or by using the ratio or difference of a received signal corresponding to two different wavelengths. Skin color can be determined by using user input like for example the user entering their skin color, an image of the person's phase, etc., and is then subsequently used to calibrate the algorithm, light source brightness, light source wavelength and the receiver gain. The effect of skin color on the raw PPG signal can also be measured in US 2016/302706 A1 by sending in a signal of known amplitude to the light sources and then measuring the received signal from the photodetector(s). This amplitude is then compared to a set of values stored in a table to determine algorithm calibration, transmitter amplitude and receiver gain.
US 2022/167883 A1 discloses an apparatus and a method for estimating a component of an analyte that includes a sensor comprising a light source configured to emit light to the analyte, and a detector configured to measure a spectrum of light reflected from the analyte, and a processor configured to : based on an initial amount of received light being obtained from the analyte by operating the sensor under initial operating conditions, determine optimal operating conditions based on the initial amount of received light and the initial operating conditions, and based on a spectrum being measured from the analyte by operating the sensor under the optimal operating conditions, estimate the component of the analyte based on the spectrum.
It is the object of the present invention to provide a device and a method that allow estimating a concentration of an antioxidant component in a very accurate manner.

This object is solved by the subject matter of the independent claims.

Preferred embodiments are defined in the dependent claims.

### SUMMARY

Provided are systems, methods and devices for accurately estimating a concentration of an antioxidant component by dynamically setting an operating condition of a sensor for estimating the concentration of the antioxidant component based on a change in skin.

Additional aspects will be set forth in part in the description which follows and, in part, will be apparent from the description, or may be learned by practice of the presented embodiments.

The invention is claimed in the independent claims. Preferred embodiments are specified in the dependent claims.

According to an aspect of the disclosure, an apparatus for estimating a concentration of a component may include a sensor including a plurality of light sources having different central wavelengths and at least one detector configured to detect light, and a processor configured to determine a first reflectance of skin using a first light source of the plurality of light sources, set an operating condition of the sensor based on the determined first reflectance, drive the plurality of light sources according to the operating condition, and estimate a concentration of an analyte component based on a plurality of light quantities detected from skin by the at least one detector.

The first light source of the plurality of light sources may have a central wavelength in a first range from about 350 nm to about 450 nm or in a second range from about 500 nm to about 600 nm.

According to the invention, the processor is configured to set an operating condition of the sensor to a first operating condition, measure a plurality of skin light quantities by driving a first light source according to the first operating condition and by using the plurality of detectors, calculate skin reflectances for each of the detectors by using the skin light quantities measured by the respective detectors and an initial light quantity, compare the skin reflectances for each of the detectors with a threshold value and determine whether there is a reflectance less than the threshold value, if all the reflectances are greater than or equal to the threshold value, maintain the current first operating condition, if there is a reflectance less than the threshold value, determine whether a percentage of the number of reflectances, which are less than the threshold value,is a predetermined percentage or more with respect to the total number of reflectances, if no, determine that only a partial area of skin is changed in color and guide a user to change a sensor measurement position, if yes, determine that the entire area of skin is changed in color and change the sensor operating condition to a second operating condition.

The processor may be further configured to set the sensor to a first operating condition, drive the first light source based on the first operating condition, determine the first reflectance based on a first light quantity detected from the skin by the at least one detector, and based on the determined first reflectance being less than a first threshold value, change the operating condition to a second operating condition.

The processor may be further configured to determine the first reflectance based on an initial light quantity and the first light quantity, where wherein the initial light quantity is detected from a standard reflector using the first light source based on the first operating condition.

The processor may be further configured to determine, as the first reflectance, a result obtained by dividing the first light quantity by the initial light quantity.

The processor may be further configured to, based on the first reflectance being greater than or equal to the first threshold value, drive a second light source of the plurality of light sources and determine a second reflectance based on a second light quantity detected from the skin by the at least one detector, and, based on the determined second reflectance being greater than a second threshold value, change the operating condition to a third operating condition.

The second light source may have a central wavelength ranging from about 450 nm to about 500 nm.

The processor may be further configured to obtain a skin spectrum based on the plurality of light quantities, determine absorbance based on the obtained skin spectrum, and estimate the concentration of the analyte component based on the determined absorbance.

The processor may be further configured to, based on a predetermined condition being satisfied, determine an operating condition of the sensor.

The operating condition of the sensor may include a drive current for driving the plurality of light sources.

The processor may be further configured to set the sensor to a first operating condition, drive the first light source based on the first operating condition, determine a plurality of reflectances based on the plurality of light quantities detected from the skin by the at least one detector, and guide a user to change a position of the sensor based on the determined plurality of reflectances.

The analyte component may include at least one of skin carotenoid, blood carotenoid, glucose, urea, lactate, triglyceride, total protein, cholesterol, and ethanol.

According to an aspect of the disclosure, a method of estimating a concentration of a component may include determining a first reflectance of skin using a first light source of a plurality of light sources, the plurality of light sources being included in a sensor and having different central wavelengths, setting an operating condition of the sensor based on the determined first reflectance, driving the plurality of light sources based on the set operating condition, and estimating a concentration of an analyte component based on a plurality of light quantities detected from the skin by at least one detector included in the sensor.

The first light source may have a central wavelength in a first range from about 350 nm to about 450 nm or in a second range from about 500 nm to about 600 nm.

According to the invention, the method comprises setting an operating condition of a sensor including a plurality of light sources and a plurality of detectors to a first operating condition; measuring a plurality of skin light quantities by driving a first light source based on the first operating condition and by using the plurality of detectors; calculating skin reflectances for each of the detectors by using the skin light quantities measured by the respective detectors and an initial light quantity; comparing the skin reflectances for each of the detectors with a threshold value and determining whether there is a reflectance less than the threshold value; if all the reflectances are greater than or equal to the threshold value, maintaining the current first operating condition; if there is a reflectance less than the threshold value, determining whether a percentage of the number of reflectances, which are less than the threshold value, is a predetermined percentage or more with respect to the total number of reflectances; if no, determining that only a partial area of skin is changed in color and guiding a user to change a sensor measurement position, if yes, determining that the entire area of skin is changed in color and changing the sensor operating condition to a second operating condition.

The setting of the operating condition of the sensor may include setting the sensor to a first operating condition, driving the first light source based on the first operating condition, determining the first reflectance based on a first light quantity detected from the skin by the at least one detector, and based on the determined first reflectance being less than a first threshold value, changing the operating condition to a second operating condition.

The determining of the first reflectance may include determining the first reflectance based on an initial light quantity and the first light quantity, where the initial light quantity is detected from a standard reflector using the first light source based on the first operating condition.

The setting of the operating condition of the sensor may include based on the first reflectance being greater than or equal to the first threshold value, driving a second light source of the plurality of light sources, determining a second reflectance based on a second light quantity detected from the skin by the at least one detector, and based on the determined second reflectance being greater than a second threshold value, changing the operating condition to a third operating condition.

The estimating of the concentration of the analyte component may include obtaining a skin spectrum based on the plurality of light quantities, determining absorbance using the obtained skin spectrum, and estimating the concentration of the analyte component based on the determined absorbance.

According to an aspect of the disclosure, a wearable device may include a main body, a sensor including a plurality of light sources having different central wavelengths and a detector configured to detect light from skin of the body of the user, and a processor configured to drive the plurality of light sources based on an operating condition of the sensor, estimate a concentration of an antioxidant component based on light detected by the detector, and based on a predetermined condition being satisfied, determine whether there is a change in color of the skin using a light source of the plurality of light sources having a predetermined central wavelength and set an operating condition of the sensor based on the determination of whether there is a change in the color of the skin.

According to the invention, the processor is configured to set an operating condition of the sensor to a first operating condition,measure a plurality of skin light quantities by driving a first light source based on the first operating condition of the sensor and by using the plurality of detectors,calculate skin reflectances for each of the detectors by using the skin light quantities measured by the respective detectors and an initial light quantity,compare the skin reflectances for each of the detectors with a threshold value and determine whether there is a reflectance less than the threshold value,if all the reflectances are greater than or equal to the threshold value, maintain the current first operating condition,if there is a reflectance less than the threshold value, determine whether a percentage of the number of reflectances, which are less than the threshold value,is a predetermined percentage or more with respect to the total number of reflectances,if no, determine that only a partial area of skin is changed in color and guide a user to change a sensor measurement position,if yes, determine that the entire area of skin is changed in color and change the sensor operating condition to a second operating condition.

The predetermined condition may be set in advance based on at least one of a period of time when the main body is not worn on the body of the user before the antioxidant component is estimated, a period of time during a day when the main body is not worn on the body of the user, and a season.

### BRIEF DESCRIPTION OF DRAWINGS

The above and other aspects, features, and advantages of certain embodiments of the present disclosure will be more apparent from the following description taken in conjunction with the accompanying drawings, in which:
FIG. 1 is a block diagram illustrating an apparatus for estimating a concentration of a component according to an embodiment of the present disclosure;
FIG. 2 is a diagram illustrating an example of a sensor structure of an apparatus for estimating a concentration of a component according to an embodiment of the present disclosure;
FIG. 3A is a diagram showing light absorption by blood components according to an embodiment of the present disclosure;
FIGS. 3B, 3C and 3D are diagrams of an example of obtaining an absorption spectrum according to an embodiment of the present disclosure;
FIG. 4 is a block diagram illustrating an apparatus for estimating a concentration of a component according to an embodiment of the present disclosure;
FIG. 5 is a diagram illustrating an example of guiding a sensor measurement position according to an embodiment of the present disclosure;
FIG. 6 is a flowchart of a method of estimating a concentration of an analyte component according to an embodiment of the present disclosure;
FIGS. 7A, 7B and 7C are flowcharts of methods of setting an operating condition based on a change in skin according to an embodiment of the present disclosure; and
FIGS. 8, 9 and 10 are diagrams illustrating examples of structures of an electronic device including an apparatus for estimating a concentration of a component according to an embodiment of the present disclosure.

Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

Details of other embodiments are included in the following detailed description and drawings. Advantages and features of the present invention, and a method of achieving the same will be more clearly understood from the following embodiments described in detail with reference to the accompanying drawings. Throughout the drawings and the detailed description, unless otherwise described, the same drawing reference numerals will be understood to refer to the same elements, features, and structures.

It will be understood that, although the terms first, second, etc. may be used herein to describe various elements, these elements should not be limited by these terms. These terms are only used to distinguish one element from another. Any references to singular may include plural unless expressly stated otherwise. In addition, unless explicitly described to the contrary, an expression such as "comprising" or "including" will be understood to imply the inclusion of stated elements but not the exclusion of any other elements. Also, the terms, such as 'unit' or 'module', etc., should be understood as a unit that performs at least one function or operation and that may be embodied as hardware, software, or a combination thereof.

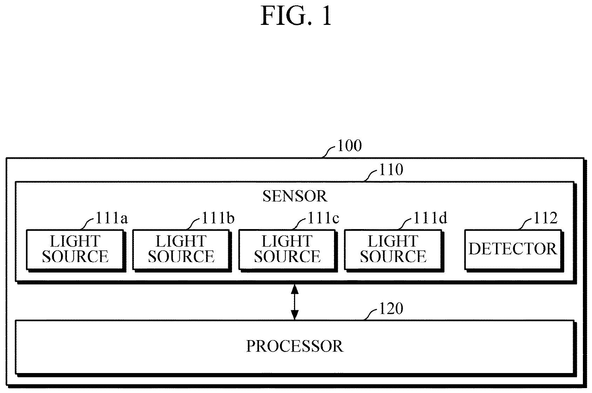

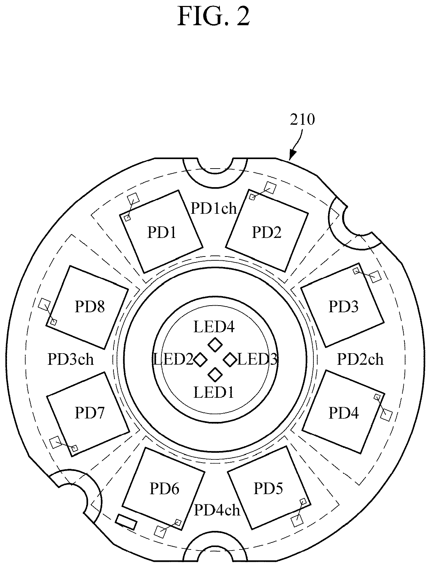

FIG. 1 is a block diagram illustrating an apparatus for estimating a concentration of an analyte component according to an embodiment of the present disclosure. FIG. 2 is a diagram illustrating an example of a sensor structure of an apparatus for estimating a concentration of a component according to an embodiment of the present disclosure.

Referring to FIG. 1, an apparatus 100 for estimating a concentration of a component includes a sensor 110 and a processor 120.

The sensor 110 may include a plurality of light sources 111a, 111b, 111c, and 111d. While four light sources 111a, 111b, 111c, and 111d are illustrated for convenience of explanation, the number of light sources is not limited thereto. Each of the light sources 111a, 111b, 111c, and 111d may be a light emitting diode (LED), a laser diode, a phosphor, etc., but the light sources are not limited to these examples. The plurality of light sources 111a, 111b, 111c, and 111d may be driven simultaneously or in a time-division manner. Alternatively, the light sources may be driven in a generally sequential manner with predetermined light sources being simultaneously driven in groups.

The respective light sources 111a, 111b, 111c, and 111d may have different central wavelengths. For example, a first light source 111a may have a central wavelength ranging from about 350 nm to about 450 nm, and a second light source 111b may have a central wavelength ranging from about 450 nm to about 500 nm. In addition, a third light source 111c and a fourth light source 111d may have central wavelengths ranging from about 500 nm to about 550 nm and ranging from about 550 nm to about 650 nm, respectively. However, the central wavelengths of the light sources are not limited thereto and may be changed according to various conditions, such as the type of analyte component, the size of a form factor of the apparatus 100, and the like.

The sensor 110 may include a detector 112 configured to detect light, convert the light into an electrical signal, and output the electrical signal. The detector 112 may include one or more photo diodes, photo transistors (PTr), etc., but is not limited thereto, and may include a complementary metal oxide semiconductor (CMOS) image sensor, a charge-coupled device (CCD) image sensor, and the like.

The sensor 110 may include an analog-digital converter configured to convert an electrical signal, output from the detector 112, into a digital signal and/or an amplifier configured to amplify the electrical signal.

Referring to FIG. 2, the sensor 110 according to an embodiment has a structure in which a plurality of LEDs (LED1 to LED4) having different central wavelengths are disposed at the center, and a plurality of photodiodes PD1 to PD8 are disposed around the periphery thereof. However, the sensor 110 is not limited thereto, and one or more photodiodes may be disposed at the center, and the plurality of LEDs (LED1 to LED4) may be disposed around the periphery thereof. In this case, each one of channels PD1ch to PD4ch include two or more of the photodiodes PD1 to PD8, such that the photodiodes may be driven on a channel basis (e.g., PD1 and PD2 may be driven on PD1ch, PD3 and PD4 may be driven on PD2ch, PD5 and PD6 may be driven on PD4ch, and PD7 and PD8 may be driven on PD3ch). The photodiodes PD1 to PD8 may be driven in synchronization with the light sources which are driven individually or on a channel basis. For example, LED1 may be synchronized with PD1ch, LED2 may be synchronized with PD2ch, LED3 may be synchronized with PD3ch, and LED4 may be synchronized with PD4ch.

Referring back to FIG. 1, the processor 120 may be electrically connected to the sensor 110. The processor 120 may set an operating condition of the sensor 110 and may control the sensor 110 according to the set operating condition. In this case, the operating condition may include parameters, such as magnitude of incident light (e.g., current intensity), gain, exposure time, aperture size, and the like. A plurality of operating conditions may be predefined by combining the parameter values, and when driving the sensor 110, the processor 120 may set one of the operating conditions as the operating condition of the sensor 110. The plurality of operating conditions may include an initial operating condition defined to be universally applied regardless of skin color, and various operating conditions defined in consideration of skin color of ordinary people or a change in skin color of an individual user.

In this case, a user's skin is a body part coming into contact with the sensor 110, and may be, for example, the palm of the hand or the sole of the foot which is covered with a thick epidermis layer, a body part that is adjacent to the radial artery or where venous blood or capillary blood passes, a peripheral part of the body with high blood vessel density, such as fingers, toes, earlobes, or the like, or the wrist and an inner part of the ear which may come into contact with a wearable device when the device is worn, and the like.

Upon receiving a user's request for estimating a concentration of an analyte component, the processor 120 may operate the sensor 110 and may estimate the concentration of the analyte component by using data obtained by the sensor 110. In this case, the analyte component may include an antioxidant component including skin carotenoid and/or blood carotenoid, or a component such as glucose, urea, lactate, triglyceride, total protein, cholesterol, ethanol, etc., but is not limited thereto. For convenience of explanation, the following description will be given using an antioxidant component as an example.

Upon receiving a request for estimating a concentration of an antioxidant component, the processor 120 may determine a change in skin color of a user and may set an operating condition suitable for estimating the concentration of the antioxidant component based on the determination, before estimating the concentration. Generally, the skin color is associated with melanin distribution, and melanin affects light absorption, thereby affecting a signal quality of reflected light. Accordingly, by properly setting an operating condition for the sensor in consideration of a change in a user's skin color, the signal quality of light acquired by the sensor 110 may be improved.

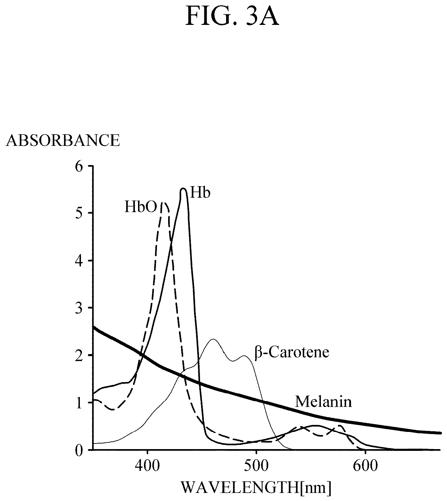

FIG. 3A is a diagram showing light absorption by blood components according to an embodiment of the present disclosure.

As illustrated herein, it can be seen that components, such as melanin, hemoglobin, and the like in blood, exhibit high light absorption in a relatively short-wavelength region (e.g., about 350 nm to about 450 nm). Particularly, light absorption by melanin gradually increases towards the short-wavelength region, such that a magnitude of reflected light in the short-wavelength region may affect a signal quality.

Accordingly, among the plurality of light sources 111a, 111b, 111c, and 111d included in the sensor 110, the processor 120 may drive the first light source 111a having a central wavelength (e.g., ranging from about 350 nm to about 450 nm according to a first operating condition) and may determine a change in skin color related to the concentration of melanin. In this case, the first operating condition may be preset by a user, and may be, for example, the aforementioned initial operating condition, a basic operating condition set by a user among a plurality of operating conditions, or an operating condition set at a most recent estimation time.

When the first light source 111a emits light to skin, and the detector 112 detects light scattered or reflected from the skin, the processor 120 may calculate a first reflectance based on a detected light quantity and may determine a change in skin color based on the calculated first reflectance. For example, the processor 120 may obtain, as the first reflectance, a result obtained by dividing the detected light quantity by an initial light quantity measured in advance for the first light source 111a according to the first operating condition. In this case, the initial light quantity may be measured in advance by using a standard reflector for each of the plurality of light sources 111a, 111b, 111c, and 111d according to each of a plurality of operating conditions. For example, the initial light quantity may be measured during the manufacture of the sensor 110 or during the first use of the apparatus 100. In this case, the standard reflector may be a reflector having a predetermined reflectance (e.g., average reflectance (about 50 %) or maximum reflectance (about 80 %) of human skin).

For example, the processor 120 may compare the calculated first reflectance with a predetermined first threshold value (e.g., about 7 %). If the first reflectance is greater than or equal to the first threshold value, the processor 120 may determine that a change in skin color is not large, and may maintain the set first operating condition (e.g., drive current of 5 mA). If the first reflectance is less than the first threshold value, the processor 120 may determine that skin color is changed, and may change the operating condition of the sensor 110 from the first operating condition (e.g., drive current of 5 mA) to a second operating condition (e.g., drive current of 20 mA). In this case, by dividing a degree, by which the first reflectance is less than the first threshold value, into a plurality of levels, the processor 120 may change the operating condition according to each level. The drive current under each operating condition may be set differently for each light source.

In another example, if the calculated first reflectance is less than the first threshold value, the processor 120 may determine that skin color is changed, and may change the operating condition to the second operating condition; and if not, the processor 120 may determine a possibility of signal saturation. The processor 120 may drive the second light source 112b, having a central wavelength in a wavelength range (e.g., about 450 nm to about 500 nm) with a relatively large magnitude of skin reflected light, among the plurality of light sources 111a, 111b, 111c, and 111d according to the first operating condition, and may determine signal saturation by using a second light quantity detected by the detector. For example, the processor 120 may obtain a second reflectance by dividing the second light quantity by an initial light quantity measured for the second light source according to the first operating condition. If the second reflectance is greater than a predetermined second threshold value, the processor 120 may determine that there is a possibility of signal saturation, and may change the operating condition of the sensor 110 to a third operating condition (e.g., drive current of 3 mA), and if not, the processor 120 may maintain the first operating condition.

While an example of determining a change in skin color by using the light source having the central wavelength ranging from about 350 nm to about 450 nm is described above, the determination is not limited thereto, and if there is no light source having the central wavelength of about 350 nm to about 450 nm, a light source having a central wavelength of about 500 nm to about 600 nm may also be used. Alternatively, a change in skin color may be determined by using a plurality of light sources having central wavelengths (e.g., ranging from about 350 nm to about 450 nm and ranging from about 500 nm to about 600 nm). For example, the processor 120 may calculate the aforementioned first reflectance by using a statistical value (e.g., sum, average, etc.) of quantities of light detected from the plurality of light sources and a statistical value (e.g., sum, average, etc.) of initial light quantities corresponding thereto.

The aforementioned process of setting the sensor operating condition based on the change in skin color may be performed if a predetermined condition is satisfied. In this case, the predetermined condition may be set so that the setting of the sensor operating condition may be performed when the apparatus 110 is first used and if there is a user's request. Alternatively, if no measurement is performed after a given period of time has elapsed, there is a possibility that skin color may change with an increasing time of exposure of skin to the sun, such that measurement may be set to be performed when a given period of time elapses after a previous measurement time. In this case, the given period of time may be set differently according to gender, age, daytime and nighttime, and/or season, and the like.

In order to determine the change in skin color, the processor 120 may drive the first light source according to the first operating condition, and may guide a user to adjust a sensor measurement position based on a plurality of light quantities detected by the plurality of detectors. For example, upon determining, based on the respective light quantities detected by the plurality of detectors, that a change occurs in the entire area or most areas of skin coming into contact with the sensor 110, the processor 120 may change the operating condition of the sensor as described above. Upon determining that only a partial area of skin has a portion which is different in color (e.g., dark spot) or is changed, the processor 120 may guide a user to change a sensor position to another position.

For example, the processor 120 may calculate reflectances for each of the plurality of detectors based on light quantities detected by the respective detectors and the initial light quantity as described above, and may compare the respective reflectances with a threshold value. If a percentage of the number of reflectances, which are less than the threshold value, is a predetermined percentage or more with respect to the total number of reflectances, the processor 120 may determine that the entire area of skin is changed in color. If a percentage of the number of reflectances, which are less than the threshold value, is not a predetermined percentage or more with respect to the total number of reflectances, the processor 120 may determine that only a partial area of skin is changed in color, and may guide a user to change a measurement position in consideration of a position of a specific detector/detector channel corresponding to the reflectance which is less than the threshold value.

The processor 120 may drive the plurality of light sources 111a, 111b, 111c, and 111d included in the sensor 110 according to the operating condition, which is set based on the above determination on the change in skin color, or according to the first operating condition if the process of determining a change in skin color is omitted, and may estimate a concentration of an antioxidant component based on the plurality of light quantities detected by the detector 112. In this case, the first operating condition may be the initial operating condition, the basic operating condition, or the operating condition set at a previous estimation time, as described above.

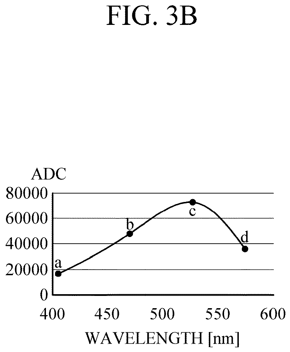

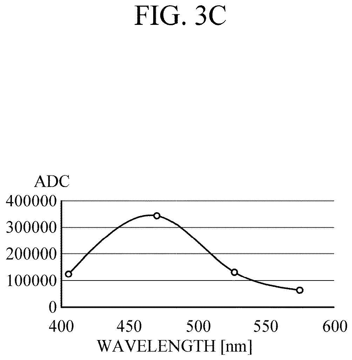

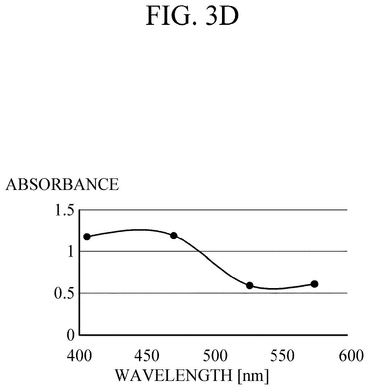

FIGS. 3B, 3C and 3D are diagrams of an example of obtaining an absorption spectrum according to an embodiment of the present disclosure. FIG. 3B is a diagram illustrating a spectrum reconstructed based on light quantities a, b, c, and d measured using the light sources 111a, 111b, 111c, and 111d having difference central wavelengths, and FIG. 3C is a diagram illustrating a calibration spectrum obtained based on the initial light quantity measured for the respective light sources 111a, 111b, 111c, and 111d by using a standard reflector. FIG. 3D is a diagram illustrating an example of a skin absorption spectrum obtained by correcting the reconstructed spectrum of FIG. 3B using the calibration spectrum of FIG. 3C.

For example, when the plurality of light sources 111a, 111b, 111c, and 111d are driven under the set operating condition and a plurality of light quantities are detected, the processor 120 may reconstruct a skin spectrum based on the detected plurality of light quantities, and may obtain an absorption spectrum based on Lambert-Beer's law by using a calibration spectrum corresponding to the set operating condition. For example, if the operating condition of the sensor 110 is changed from the first operating condition to the second operating condition, the processor 120 may obtain a skin absorption spectrum by using the calibration spectrum obtained using the standard reflector according to the second operating condition.

Upon obtaining the skin absorption spectrum, the processor 120 may estimate a concentration of an antioxidant component by using the skin absorption spectrum. For example, the processor 120 may estimate a concentration of an analyte component by applying a predefined estimation model to the obtained absorbance. In this case, the estimation model may be defined in advance as, for example, linear combination equation, but is not limited thereto.

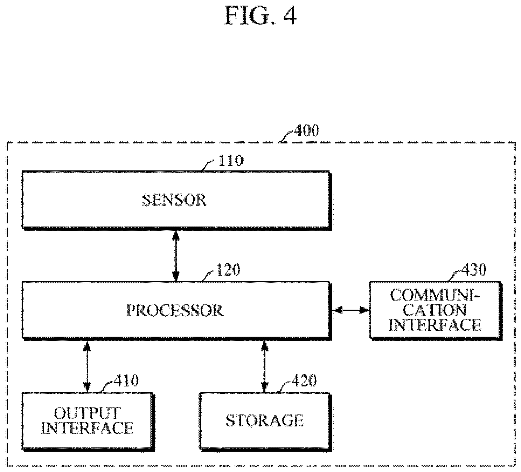

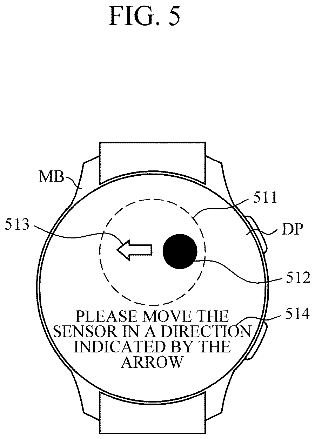

FIG. 4 is a block diagram illustrating an apparatus for estimating a concentration of a component according to an embodiment of the present disclosure. FIG. 5 is a diagram illustrating an example of guiding a sensor measurement position according to an embodiment of the present disclosure.

Referring to FIG. 4, an apparatus 400 for estimating a component includes the sensor 110, the processor 120, an output interface 410, a storage 420, and a communication interface 430. The sensor 110 may include a plurality of light sources and detectors. The sensor 110 and the processor 120 are described above, such that a detailed description thereof will be omitted.

The output interface 410 may output data processed by the processor 120. For example, the output interface 410 may output information, such as spectrum data, a result of estimating a concentration of an analyte component, sensor operating conditions, and the like. The output interface 410 may include a visual output module such as a display, an audio output module such as a speaker, or a haptic module using vibrations, tactile sensation, and the like. For example, the output interface 410 may output information, such as the spectrum data, analyte component concentration estimation data, sensor operating conditions, and the like.

In addition, the output interface 410 may output information for guiding a user to adjust a sensor measurement position generated by the processor 120. As illustrated in FIG. 5, the output interface 410 may output, to a display DP of a main body MB, a graphic object 511 (e.g., a circular box, a square box, skin image, etc.), which indicates a skin area 511 coming into contact with the sensor 110, an object 512 indicating a position at which skin color is different, a circular box or an image. In this case, the depth of the color of the object 512 may be set differently according to a degree of color change (e.g., degree of difference between a threshold value and reflectance). In addition, the output interface 410 may output an object 513, such as an arrow indicating a moving direction, and/or a text message 514, such as "please move the sensor in a direction indicated by the arrow," in order to move a sensor position.

The storage 420 may store data related to estimating a component concentration. For example, the storage 420 may store user characteristic information, such as a user's age, gender, health condition, etc., various sensor operating conditions, initial light quantity, component estimation model, and the like. Further, the storage 420 may store information, such as an estimated component concentration value generated by the processor 120, and the like.

The storage 420 may include at least one storage medium of a flash memory type memory, a hard disk type memory, a multimedia card micro type memory, a card type memory (e.g., a secure digital (SD) memory, an XD memory, etc.), a random access memory (RAM), a static random access memory (SRAM), a read only memory (ROM), an electrically erasable programmable ROM (EEPROM), a programmable ROM (PROM), a magnetic memory, a magnetic disk, and an optical disk, and the like, but is not limited thereto.

The communication interface 430 may communicate with an external device by wired or wireless communications to receive various data related to estimating a component. The external device may include an information processing device such as a smartphone, a tablet PC, a laptop computer, a desktop computer, etc., but is not limited thereto. The communication interface 430 may communicate with the external device by using communication techniques, such as Bluetooth communication, Bluetooth low energy (BLE) communication, near field communication (NFC), wireless local access network (WLAN) communication, Zigbee communication, infrared data association (IrDA) communication, Wi-Fi direct (WFD) communication, ultra-wideband (UWB) communication, Ant+ communication, Wi-Fi communication, radio frequency identification (RFID) communication, 3^{rd} generation (3G), 4^{th} generation (4G), and 5^{th} generation (5G) communications, and the like. However, this is merely exemplary and is not intended to be limiting.

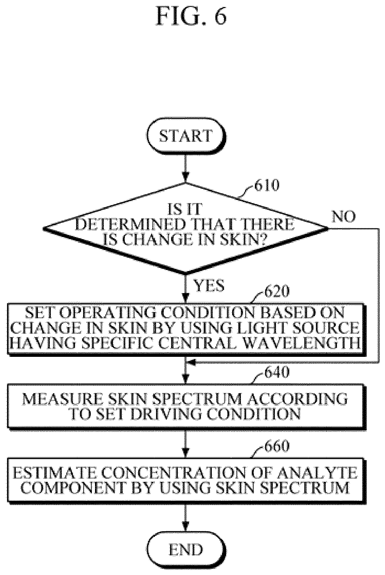

FIG. 6 is a flowchart of a method of estimating a concentration of an analyte component according to an embodiment of the present disclosure. The method of estimating a concentration of an analyte component may be performed by the aforementioned apparatuses 100 and 400 for estimating a concentration of a component, which are described in detail above, and thus will be briefly described below.

Referring to FIG. 6A, in response to a request for estimating a concentration of an analyte component, in operation 610, the apparatus for estimating a concentration of a component may determine whether there is a change in skin color by using one light source having a specific central wavelength among the plurality of light sources included in the sensor. Based on determining that there is no change in skin, the apparatus may proceed to operation 640. Based on determining that there is a change in skin, in operation 620, the apparatus may set a sensor operating condition suitable for estimating a component concentration. In this case, the plurality of light sources may have different central wavelengths.

In an embodiment, in operation 610 or prior to operation 610, in response to the request for estimating the concentration of the analyte component, the apparatus for estimating a concentration of a component may first determine whether a predetermined condition is satisfied prior to operation 610, and if the predetermined condition is satisfied, the apparatus for estimating a concentration of a component may determine whether there is a change in skin in operation 610 and may set a sensor operating condition based on the skin change in operation 620. In this case, the predetermined condition may be set such that the determining whether there is a change in skin may be performed at the first use of the apparatus, at predetermined intervals, or if measurement with the sensor is not performed after a predetermined period of time or more has elapsed.

Then, the apparatus for estimating a concentration of a component may measure a skin spectrum by driving the plurality of light sources and detectors according to the set sensor operating condition in operation 640. The apparatus for estimating a concentration of a component may reconstruct the spectrum by driving the plurality of light sources according to the set sensor operating condition and by using light quantities detected for the respective light sources, and may generate a skin absorption spectrum based on a calibration spectrum measured using a standard reflector according to the set sensor operating condition.

Subsequently, the apparatus for estimating a concentration of a component may estimate the concentration of the analyte component by using the skin spectrum in operation 660. For example, the apparatus for estimating a concentration of a component may estimate the concentration of the component by applying a predefined component estimation model to absorbances obtained for the respective light sources.

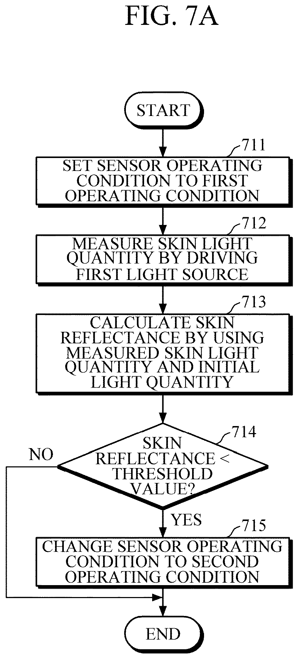

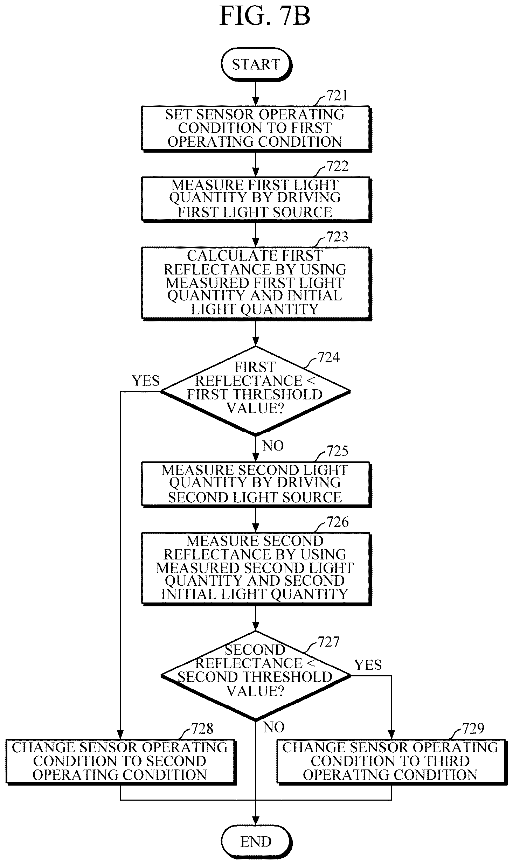

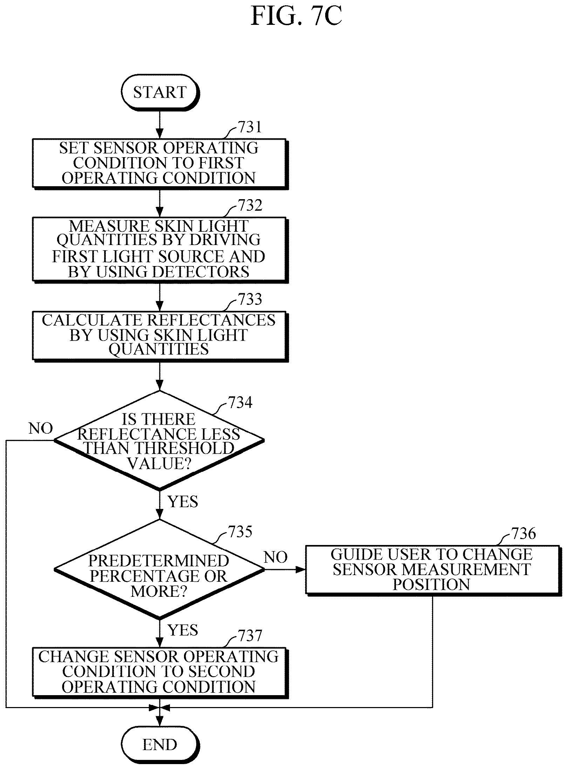

FIGS. 7A, 7B and 7C are flowcharts of methods of setting an operating condition based on a change in skin according to an embodiment of the present disclosure. FIGS. 7A to 7C are diagrams illustrating various examples of setting the sensor operating condition in 620 of FIG. 6.

Referring to FIG. 7A, the apparatus for estimating a concentration of a component may set the sensor operating condition to a first operating condition in operation 711. The first operating condition may be an initial operating condition, a basic operating condition set by a user, or an operating condition set at a previous estimation time, and may maintain an operating condition set at the previous measurement time according to, for example, a user's setting, or may reset the operating condition to the initial operating condition or the basic operating condition.

Then, by driving the first light source according to the set first operating condition, the apparatus for estimating a concentration of a component may measure a skin light quantity by using the detector in operation 712. In this case, the first light source may be a light source having a central wavelength of about 350 nm to about 450 nm. However, the light source is not limited thereto, and a light source having a central wavelength of about 500 nm to about 600 nm may also be used as needed, and a plurality of light sources having central wavelengths in a range of about 350 nm to about 450 nm and a range of about 500 nm to about 600 nm may also be used.

Subsequently, the apparatus for estimating a concentration of a component may calculate skin reflectance in operation 713 by using the light quantity measured in operation 712 and the initial light quantity. In this case, the initial light quantity may be a quantity of reflected light which is measured using a standard reflector by driving the first light source according to the first operating condition.

Next, the apparatus for estimating a concentration of a component may compare the calculated skin reflectance with a threshold value in operation 714. Upon comparison, if the skin reflectance is greater than or equal to the threshold value, the apparatus for estimating a concentration of a component may maintain the current first operating condition, and if the skin reflectance is less than the threshold value, the apparatus for estimating a concentration of a component may change the sensor operating condition to the second operating condition in operation 715.

Referring to FIG. 7B, the apparatus for estimating a concentration of a component may first set the sensor operating condition to the first operating condition in operation 721. The first operating condition may be an initial operating condition, a basic operating condition set by a user, or an operating condition set at a previous estimation time.

Then, by driving the first light source according to the set first operating condition, the apparatus for estimating a concentration of a component may measure a first light quantity by using the detector in operation 722. In this case, the first light source may be a light source having a central wavelength of about 350 nm to about 450 nm. However, the light source is not limited thereto, and a light source having a central wavelength of about 500 nm to about 600 nm may also be used as needed, and a plurality of light sources having central wavelengths in a range of about 350 nm to about 450 nm and a range of about 500 nm to about 600 nm may also be used.

Subsequently, the apparatus for estimating a concentration of a component may calculate a first reflectance in operation 723 by using the first light quantity measured in operation 722 and the initial light quantity. In this case, the initial light quantity may be a quantity of reflected light which is measured using a standard reflector by driving the first light source according to the first operating condition.

Next, the apparatus for estimating a concentration of a component may compare the calculated first reflectance with a first threshold value in operation 724. Upon comparison, if the first reflectance is less than the threshold value, the apparatus for estimating a concentration of a component may change the sensor operating condition to the second operating condition in operation 728. Upon comparison, if the first reflectance is greater than or equal to the threshold value, the apparatus for estimating a concentration of a component may drive the second light source to measure a second light quantity in operation 725. In this case, the second light source may be a light source having a central wavelength of about 450 nm to about 500 nm.

Then, the apparatus for estimating a concentration of a component may measure a second reflectance by using the measured second light quantity and a second initial light quantity in operation 726. In this case, the second initial light quantity may be a quantity of reflected light which is measured using a standard reflector by driving the second light source according to the first operating condition.

Subsequently, the apparatus for estimating a concentration of a component may compare the second reflectance with a second threshold value in operation 727, and if the second reflectance is less than or equal to the second threshold value, the apparatus for estimating a concentration of a component may maintain the current first operating condition, and if the second reflectance is greater than the second threshold value, the apparatus for estimating a concentration of a component may change the sensor operating condition to a third operating condition in operation 729.

Referring to FIG. 7C, the apparatus for estimating a concentration of a component may set the sensor operating condition to the first operating condition in operation 731.

Then, the apparatus for estimating a concentration of a component may measure a plurality of skin light quantities by driving the first light source according to the first operating condition and by using the plurality of detectors in operation 732.

Subsequently, the apparatus for estimating a concentration of a component may calculate skin reflectances in operation 733 for each of the detectors by using the light quantities measured in operation 732 by the respective detectors and the initial light quantity.

Next, the apparatus for estimating a concentration of a component may compare the skin reflectances, calculated for each of the detectors, with a threshold value and may determine whether there is a reflectance less than the threshold value in operation 734. Upon comparison, if all the reflectances are greater than or equal to the threshold value, the apparatus for estimating a concentration of a component may maintain the current first operating condition.

Upon comparison, if there is a reflectance less than the threshold value, the apparatus for estimating a concentration of a component may determine whether a percentage of the number of reflectances, which are less than the threshold value, is a predetermined percentage or more with respect to the total number of reflectances in operation 735. If the percentage of the number of reflectances is not the predetermined percentage or more with respect to the total number of reflectances, the apparatus for estimating a concentration of a component may determine that only a partial area of skin is changed in color and may guide a user to change a sensor measurement position in operation 736. If the percentage of the number of reflectances, which are less than the threshold value, is the predetermined percentage or more with respect to the total number of reflectances, the apparatus for estimating a concentration of a component may determine that the entire area of skin is changed in color and may change the sensor operating condition to the second operating condition in operation 737.

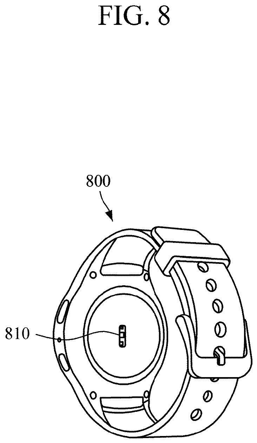

FIGS. 8, 9 and 10 are diagrams illustrating examples of structures of an electronic device including an apparatus for estimating a concentration of a component according to an embodiment of the present disclosure. FIGS. 8 to 10 are diagrams illustrating examples of various structures of an electronic device including the apparatuses 100 and 400 for estimating a concentration of a component.

The electronic device may include, for example, various types of wearable devices (e.g., a smart watch, a smart band, smart glasses, smart earphones, a smart ring, a smart patch, and a smart necklace, and a mobile device such as a smartphone, a tablet PC, etc.), or home appliances or various Internet of Things (IoT) devices (e.g., home IoT device, etc.) based on IoT technology.

The electronic device may include a sensor device, a processor, an input device, a communication module, a camera module, an output device, a storage device, and a power module. All the components of the electronic device may be integrally mounted in a specific device or may be distributed in two or more devices. The sensor device may include the sensor 110 of the apparatuses 100 and 400 for estimating a concentration of a component, and may further include an additional sensor, such as a gyro sensor, a global positioning system (GPS), and the like.

The processor may execute programs, stored in the storage device, to control components connected to the processor, and may perform various data processing or computation, including estimation of bio-information. The processor may include a main processor (e.g., a central processing unit (CPU) or an application processor (AP), etc.), and an auxiliary processor (e.g., a graphics processing unit (GPU), an image signal processor (ISP), a sensor hub processor, or a communication processor (CP), etc.), which is operable independently from, or in conjunction with, the main processor.

The input device may receive a command and/or data to be used by each component of the electronic device, from a user and the like. The input device may include, for example, a microphone, a mouse, a keyboard, or a digital pen (e.g., a stylus pen, etc.).

The communication module may support establishment of a direct (e.g., wired) communication channel and/or a wireless communication channel between the electronic device and other electronic device, a server, or the sensor device within a network environment, and performing of communication via the established communication channel. The communication module may include one or more communication processors that are operable independently from the processor and supports a direct communication and/or a wireless communication. The communication module may include a wireless communication module (e.g., a cellular communication module, a short-range wireless communication module, or a global navigation satellite system (GNSS) communication module, etc.), and/or a wired communication module (e.g., a local area network (LAN) communication module, a power line communication (PLC) module, and the like). These various types of communication modules may be integrated into a single chip, or may be separately implemented as multiple chips. The wireless communication module may identify and authenticate the electronic device in a communication network by using subscriber information (e.g., international mobile subscriber identity (IMSI), etc.) stored in a subscriber identification module.

The camera module may capture still images or moving images. The camera module may include a lens assembly having one or more lenses, image sensors, image signal processors, and/or flashes. The lens assembly included in the camera module may collect light emanating from a subject to be imaged.

The output device may visually/non-visually output data generated or processed by the electronic device. The output device may include a sound output device, a display device, an audio module, and/or a haptic module.

The sound output device may output sound signals to the outside of the electronic device. The sound output device may include a speaker and/or a receiver. The speaker may be used for general purposes, such as playing multimedia or playing record, and the receiver may be used for incoming calls. The receiver may be implemented separately from, or as part of, the speaker.

The display device may visually provide information to the outside of the electronic device. The display device may include, for example, a display, a hologram device, or a projector and control circuitry to control the devices. The display device may include touch circuitry adapted to detect a touch, and/or sensor circuitry (e.g., pressure sensor, etc.) adapted to measure the intensity of force incurred by the touch.

The audio module may convert a sound into an electrical signal or vice versa. The audio module may obtain the sound via the input device, or may output the sound via the sound output device, and/or a speaker and/or a headphone of another electronic device directly or wirelessly connected to the electronic device.

The haptic module may convert an electrical signal into a mechanical stimulus (e.g., vibration, motion, etc.) or electrical stimulus which may be recognized by a user by tactile sensation or kinesthetic sensation. The haptic module may include, for example, a motor, a piezoelectric element, and/or an electric stimulator.

The storage device may store operating conditions required for operating the sensor device, and various data necessary for other components of the electronic device. The various data may include, for example, input data and/or output data for software and instructions associated with the software, and the like. The storage device may include a volatile memory and/or a non-volatile memory.

The power module may manage power supplied to the electronic device. The power module may be implemented as part of, for example, a power management integrated circuit (PMIC). The power module may include a battery, which may include a primary cell which is not rechargeable, a secondary cell which is rechargeable, and/or a fuel cell.

Referring to FIG. 8, the electronic device may be implemented as a wristwatch wearable device 800, and may include a main body and a wrist strap. A display is provided on a front surface of the main body, and may display various application screens, including an estimated analyte component concentration value, warning information, time information, received message information, and the like. A sensor device 810 may be disposed on a rear surface of the main body. During estimation of a concentration of an analyte component, the wearable device 800 may first determine whether there is a change in skin color and may basically perform an operation of resetting a sensor operating condition based on the determination, or may check a predetermined condition based on a period of time when the wearable device 800 is not worn on the wrist, season, daytime or nighttime, etc., and if the predetermined condition is satisfied, the wearable device 800 may perform the operation of resetting the sensor operating condition.

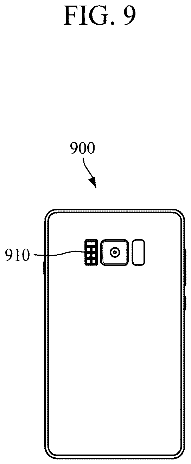

Referring to FIG. 9, the electronic device may be implemented as a mobile device 900 such as a smartphone. The mobile device 900 may include a housing and a display panel. The housing may form an exterior of the mobile device 900. The housing has a first surface, on which a display panel and a cover glass may be disposed sequentially, and the display panel may be exposed to the outside through the cover glass. A sensor device 910, a camera module and/or an infrared sensor, and the like may be disposed on a second surface of the housing. The processor and various other components may be disposed in the housing.

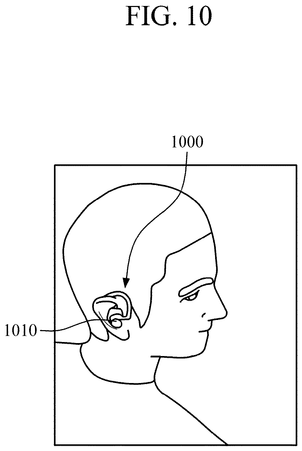

Referring to FIG. 10, the electronic device may be implemented as an ear-wearable device 1000. The ear-wearable device 1000 may include a main body and an ear strap. A user may wear the ear-wearable device 1000 by hanging the ear strap on the auricle. The ear strap may be omitted depending on a shape of the ear-wearable device 1000. The main body may be inserted into the external auditory meatus. A sensor device 1010 may be mounted in the main body at a portion coming into contact with skin. Further, a processor, a communication device, and the like may be mounted in the main body. The processor may estimate a concentration of a component based on light measured by the sensor 1010 as described above, and may transmit an estimation result to another electronic device through the communication device. Alternatively, the processor may transmit light data, measured by the sensor 1010, to another electronic device so that the electronic device may estimate the component concentration.

The present disclosure can be realized as a computer-readable code written on a computer-readable recording/storage medium. The computer-readable recording/storage medium may be any type of recording or storage device in which data is stored in a computer-readable manner.

Examples of the computer-readable recording/storage medium include a ROM, a RAM, a compact disc (CD) ROM (CD-ROM), a magnetic tape, a floppy disc, an optical data storage, and a carrier wave (e.g., data transmission through the Internet). The computer-readable recording/storage medium can be distributed over a plurality of computer systems connected to a network so that a computer-readable code is written thereto and executed therefrom in a decentralized manner. Functional programs, codes, and code segments needed for realizing the present invention can be readily deduced by programmers of ordinary skill in the art to which the invention pertains.

The disclosure has been described herein with regard to preferred embodiments. However, it will be obvious to those skilled in the art that various changes and modifications can be made without changing technical conception and essential features of the present disclosure. Thus, it is clear that the above-described embodiments are illustrative in all aspects and are not intended to limit the present disclosure.

## Claims

1. A computer-readable storage medium having instructions that, when performed by a processor (120) of an apparatus (100) for estimating a concentration of a component comprising a sensor (110) that comprises a plurality of light sources (111a, 111b, 111c, 111d) having different central wavelengths, and a plurality of detectors (112) configured to detect light, cause the processor (120) to:
set (731) an operating condition of the sensor (110) to a first operating condition,
measure (732) a plurality of skin light quantities by driving a first light source (111a) according to the first operating condition and by using the plurality of detectors (112),
calculate (733) skin reflectances for each of the detectors (112) by using the skin light quantities measured by the respective detectors (112) and an initial light quantity,
compare (734) the skin reflectances for each of the detectors (112) with a threshold value and determine whether there is a reflectance less than the threshold value,
if all the reflectances are greater than or equal to the threshold value, maintain the current first operating condition,
if there is a reflectance less than the threshold value, determine (735) whether a percentage of the number of reflectances, which are less than the threshold value,
is a predetermined percentage or more with respect to the total number of reflectances,
if no, determine that only a partial area of skin is changed in color and guide (736) a user to change a sensor measurement position,
if yes, determine that the entire area of skin is changed in color and change (737) the sensor operating condition to a second operating condition.

2. The computer-readable storage medium of claim 1, wherein a first light source (111a) of the plurality of light sources (111a, 111b, 111c, 111d) has a central wavelength in a first range from about 350 nm to about 450 nm or in a second range from about 500 nm to about 600 nm.

3. The computer-readable storage medium of claim 1 or 2,
wherein the initial light quantity is detected from a standard reflector using the first light source (111a) based on the first operating condition, and
wherein the processor (120) is further configured to determine, as a first reflectance, a result obtained by dividing a first light quantity by the initial light quantity.

4. The computer-readable storage medium of one of claims 1 to 3, wherein the processor (120) is further configured to:
obtain (640) a skin spectrum based on the plurality of light quantities,
determine absorbance based on the obtained skin spectrum, and
estimate (660) a concentration of an analyte component based on the determined absorbance.

5. The computer-readable storage medium of one of claims 1 to 4, wherein the processor (120) is further configured to, based on a predetermined condition being satisfied, determine the operating condition of the sensor (110), and/or
wherein the operating condition of the sensor (110) comprises a drive current for driving the plurality of light sources (111a, 111b, 111c, 111d).

6. The computer-readable storage medium of one of claims 1 to 5, wherein the analyte component comprises at least one of skin carotenoid, blood carotenoid, glucose, urea, lactate, triglyceride, total protein, cholesterol, and ethanol.

7. A method of estimating a concentration of a component, the method comprising:
setting (731) an operating condition of a sensor (110) including a plurality of light sources (111a, 111b, 111c, 111d) and a plurality of detectors (112) to a first operating condition;
measuring (732) a plurality of skin light quantities by driving a first light source (111a) based on the first operating condition and by using the plurality of detectors (112);
calculating (733) skin reflectances for each of the detectors (112) by using the skin light quantities measured by the respective detectors (112) and an initial light quantity;
comparing the skin reflectances for each of the detectors (112) with a threshold value and determining (734) whether there is a reflectance less than the threshold value;
if all the reflectances are greater than or equal to the threshold value, maintaining the current first operating condition;
if there is a reflectance less than the threshold value, determining (735) whether a percentage of the number of reflectances, which are less than the threshold value, is a predetermined percentage or more with respect to the total number of reflectances;
if no, determining that only a partial area of skin is changed in color and guiding (736) a user to change a sensor measurement position,
if yes, determining that the entire area of skin is changed in color and changing (737) the sensor operating condition to a second operating condition.

8. The method of claim 7, wherein the initial light quantity is detected from a standard reflector using a first light source (111a) based on the first operating condition.

9. The method of claim 7 or 8, further comprising estimating aconcentration of an analyte component, including:
obtaining (620) a skin spectrum based on the plurality of light quantities;
determining absorbance using the obtained skin spectrum; and
estimating (660) the concentration of the analyte component based on the determined absorbance.

10. A wearable device (800) comprising:
a main body worn on a body of a user;
a sensor (110, 810) comprising:
a plurality of light sources (111a, 111b, 111c, 111d) having different central wavelengths, and
a plurality of detectors (112) configured to detect light from skin of the body of the user; and
a processor (120) configured to:
set (731) an operating condition of the sensor (110, 810) to a first operating condition,
measure (732) a plurality of skin light quantities by driving a first light source (111a) based on the first operating condition of the sensor (110, 810) and by using the plurality of detectors (112),
calculate (733) skin reflectances for each of the detectors (112) by using the skin light quantities measured by the respective detectors (112) and an initial light quantity,
compare (734) the skin reflectances for each of the detectors (112) with a threshold value and determine whether there is a reflectance less than the threshold value,
if all the reflectances are greater than or equal to the threshold value, maintain the current first operating condition,
if there is a reflectance less than the threshold value, determine (735) whether a percentage of the number of reflectances, which are less than the threshold value, is a predetermined percentage or more with respect to the total number of reflectances,
if no, determine that only a partial area of skin is changed in color and guide (736) a user to change a sensor measurement position,
if yes, determine that the entire area of skin is changed in color and change (737) the sensor operating condition to a second operating condition.

11. The wearable device (800) of claim 10, wherein the first operating condition is set in advance based on at least one of:
a period of time when the main body is not worn on the body of the user before an antioxidant component is estimated,
a period of time during a day when the main body is not worn on the body of the user, and
a season.

## Patentansprüche

1. Computerlesbares Speichermedium, das Befehle aufweist, die, wenn sie von einem Prozessor (120) einer Vorrichtung (100) zum Schätzen einer Konzentration einer Komponente, die einen Sensor (110), der eine Vielzahl von Lichtquellen (111a, 111b, 111c, 111d) mit unterschiedlichen Mittenwellenlängen umfasst, sowie eine Vielzahl von Detektoren (112) umfasst, die zum Erfassen von Licht ausgeführt sind, den Prozessor (120) veranlassen zum:
Einstellen (731) eines Betriebszustandes des Sensors (110) auf einen ersten Betriebszustand,
Messen (732) einer Vielzahl von Haut-Lichtmengen durch Ansteuern einer ersten Lichtquelle (111a) entsprechend dem ersten Betriebszustand und unter Einsatz der Vielzahl von Detektoren (112),
Berechnen (733) von Haut-Reflexionsgraden für jeden der Detektoren (112) unter Verwendung der durch die entsprechenden Detektoren (112) gemessenen Haut-Lichtmengen sowie einer Anfangs-Lichtmenge,
Vergleichen (734) der Haut-Reflexionsgrade für jeden der Detektoren (112) mit einem Schwellenwert und Feststellen, ob ein Reflexionsgrad unter dem Schwellenwert liegt,
wenn alle der Reflexionsgrade über oder auf dem Schwellenwert liegen, Beibehalten des aktuellen ersten Betriebszustandes,
Feststellen (735), ob ein Prozentsatz der Anzahl von Reflexionsgraden, die unter dem Schwellenwert liegen, ein vorgegebener oder größerer Prozentsatz in Bezug auf die Gesamtzahl von Reflexionsgraden ist, wenn ein Reflexionsgrad unter dem Schwellenwert liegt,
Feststellen, dass sich nur für einen Teilbereich der Haut die Farbe geändert hat, und Führen (736) eines Benutzers beim Ändern einer Sensor-Messposition, wenn dies nicht der Fall ist,
Feststellen, dass sich für den gesamten Hautbereich die Farbe geändert hat, und Ändern (737) des Sensor-Betriebszustandes auf einen zweiten Betriebszustand, wenn dies der Fall ist.

2. Computerlesbares Speichermedium nach Anspruch 1, wobei eine erste Lichtquelle (111a) der Vielzahl von Lichtquellen (111a, 111b, 111c, 111 d) eine Mittenwellenlänge in einem ersten Bereich von ungefähr 350 nm bis ungefähr 450 nm oder in einem zweiten Bereich von ungefähr 500 nm bis ungefähr 600 nm hat.

3. Computerlesbares Speichermedium nach Anspruch 1 oder 2,
wobei die Anfangs-Lichtmenge von einem Standard-Reflektor unter Verwendung der ersten Lichtquelle (111a) auf Basis des ersten Betriebszustandes erfasst wird, und wobei der Prozessor (120) des Weiteren so ausgeführt ist, dass er als einen ersten Reflexionsgrad ein Ergebnis bestimmt, das ermittelt wird, indem eine erste Lichtmenge durch die Anfangs-Lichtmenge dividiert wird.

4. Computerlesbares Speichermedium nach einem der Ansprüche 1 bis 3, wobei der Prozessor (120) des Weiteren ausgeführt ist zum:
Ermitteln (640) eines Haut-Spektrums auf Basis der Vielzahl von Lichtmengen,
Bestimmen eines Absorptionsgrades auf Basis des ermittelten Haut-Spektrums, sowie Schätzen (660) einer Konzentration einer Analyt-Komponente auf Basis des bestimmten Absorptionsgrades.

5. Computerlesbares Speichermedium nach einem der Ansprüche 1 bis 4, wobei der Prozessor (120) des Weiteren so ausgeführt ist, dass er, basierend darauf, dass eine vorgegebene Bedingung erfüllt ist, den Betriebszustand des Sensors (110) bestimmt, und/oder
wobei der Betriebszustand des Sensors (110) einen Ansteuerstrom zum Ansteuern der Vielzahl von Lichtquellen (111a, 111b, 111c, 111d) umfasst.

6. Computerlesbares Speichermedium nach einem der Ansprüche 1 bis 5, wobei die Analyt-Komponente Haut-Carotinoid, Blut-Carotinoid, Glukose, Harnstoff, Lactat, Triglycerid, Gesamtprotein, Cholesterin oder/und Ethanol umfasst.

7. Verfahren zum Schätzen einer Konzentration einer Komponente, wobei das Verfahren umfasst:
Einstellen (730) eines Betriebszustandes eines Sensors (110), der eine Vielzahl von Lichtquellen (111a, 111b, 111c, 111d) sowie eine Vielzahl von Detektoren (112) enthält, auf einen ersten Betriebszustand;
Messen (732) einer Vielzahl von Haut-Lichtmengen durch Ansteuern einer ersten Lichtquelle (111a) auf Basis des ersten Betriebszustandes und unter Einsatz der Vielzahl von Detektoren (112);
Berechnen (733) von Haut-Reflexionsgraden für jeden der Detektoren (112) unter Verwendung der durch die entsprechenden Detektoren (112) gemessenen Haut-Lichtmengen sowie einer Anfangs-Lichtmenge,
Vergleichen der Haut-Reflexionsgrade für jeden der Detektoren (112) mit einem Schwellenwert und Feststellen (734), ob ein Reflexionsgrad unter dem Schwellenwert liegt;
Beibehalten des aktuellen ersten Betriebszustandes, wenn alle der Reflexionsgrade über oder auf dem Schwellenwert liegen;
Feststellen (735), ob ein Prozentsatz der Anzahl von Reflexionsgraden, die unter dem Schwellenwert liegen, ein vorgegebener oder größerer Prozentsatz in Bezug auf die Gesamtzahl von Reflexionsgraden ist, wenn ein Reflexionsgrad unter dem Schwellenwert liegt;
Feststellen, dass sich nur für einen Teilbereich der Haut die Farbe geändert hat, und Führen (736) eines Benutzers beim Ändern einer Sensor-Messposition, wenn dies nicht der Fall ist,
Feststellen, dass sich für den gesamten Hautbereich die Farbe geändert hat, und Ändern (737) des Sensor-Betriebszustandes auf einen zweiten Betriebszustand, wenn dies der Fall ist.

8. Verfahren nach Anspruch 7, wobei die Anfangs-Lichtmenge von einem Standard-Reflektor unter Verwendung einer ersten Lichtquelle (111a) auf Basis des ersten Betriebszustandes erfasst wird.

9. Verfahren nach Anspruch 7 oder 8, das des Weiteren Schätzen einer Konzentration einer Analyt-Komponente umfasst ,das einschließt:
Ermitteln (620) eines Haut-Spektrums auf Basis der Vielzahl von Lichtmengen;
Bestimmen eines Absorptionsgrades unter Verwendung des ermittelten Haut-Spektrums; sowie
Schätzen (660) der Konzentration der Analyt-Komponente auf Basis des bestimmten Absorptionsgrades.

10. Tragbare Vorrichtung (800), die umfasst:
einen Hauptkörper, der an einem Körper eines Benutzers getragen wird;
einen Sensor (110, 810), der umfasst:
eine Vielzahl von Lichtquellen (111a, 111b, 111c, 111d) mit unterschiedlichen Mittenwellenlängen, sowie
eine Vielzahl von Detektoren (112), die zum Erfassen von Licht von Haut des Körpers des Benutzers ausgeführt sind; und
einen Prozessor (120), der ausgeführt ist zum:
Einstellen (731) eines Betriebszustandes des Sensors (110, 810) auf einen ersten Betriebszustand,
Messen (732) einer Vielzahl von Haut-Lichtmengen durch Ansteuern einer ersten Lichtquelle (111a) auf Basis des ersten Betriebszustandes des Sensors (110, 810) und unter Einsatz der Vielzahl von Detektoren (112), Berechnen (733) von Haut-Reflexionsgraden für jeden der Detektoren (112) unter Verwendung der durch die entsprechenden Detektoren (112) gemessenen Haut-Lichtmengen sowie einer Anfangs-Lichtmenge,
Vergleichen (734) der Haut-Reflexionsgrade für jeden der Detektoren (112) mit einem Schwellenwert und Feststellen, ob ein Reflexionsgrad unter dem Schwellenwert liegt,
wenn alle der Reflexionsgrade über oder auf dem Schwellenwert liegen, Beibehalten des aktuellen ersten Betriebszustandes,
Feststellen (735), ob ein Prozentsatz der Anzahl von Reflexionsgraden, die unter dem Schwellenwert liegen, ein vorgegebener oder größerer Prozentsatz in Bezug auf die Gesamtzahl von Reflexionsgraden ist, wenn ein Reflexionsgrad unter dem Schwellenwert liegt,
Feststellen, dass sich nur für einen Teilbereich der Haut die Farbe geändert hat, und Führen (736) eines Benutzers beim Ändern einer Sensor-Messposition, wenn dies nicht der Fall ist,
Feststellen, dass sich für den gesamten Hautbereich die Farbe geändert hat, und Ändern (737) des Sensor-Betriebszustandes auf einen zweiten Betriebszustand, wenn dies der Fall ist.

11. Tragbare Vorrichtung (800) nach Anspruch 10, wobei der erste Betriebszustand im Voraus eingestellt wird basierend auf:
einem Zeitraum, in dem der Hauptkörper nicht an dem Körper des Benutzers getragen wird, bevor eine Antioxidans-Komponente geschätzt wird,
einem Zeitraum während eines Tages, in dem der Hauptkörper nicht an dem Körper des Benutzers getragen wird, oder/und
einer Jahreszeit.

## Revendications

1. Support de stockage lisible par ordinateur ayant des instructions qui, lorsqu'elles sont effectuées par un processeur (120) d'un appareil (100) destiné à estimer une concentration d'un composant comprenant un capteur (110) qui comprend une pluralité de sources lumineuses (111a, 111b, 111c, 111d) ayant différentes longueurs d'onde centrales, et une pluralité de détecteurs (112) configurés pour détecter de la lumière, amènent le processeur (120) à :
établir (731) une condition de fonctionnement du capteur (110) à une première condition de fonctionnement,
mesurer (732) une pluralité de quantités de lumière cutanées en entraînant une première source lumineuse (111a) selon la première condition de fonctionnement et en utilisant la pluralité de détecteurs (112),
calculer (733) des réflectances cutanées pour chacun des détecteurs (112) en utilisant les quantités de lumière cutanées mesurées par les détecteurs respectifs (112) et une quantité de lumière initiale,
comparer (734) les réflectances cutanées pour chacun des détecteurs (112) à une valeur seuil et déterminer s'il existe une réflectance inférieure à la valeur seuil,
si toutes les réflectances sont supérieures ou égales à la valeur seuil, maintenir la première condition de fonctionnement courante,
s'il existe une réflectance inférieure à la valeur seuil, déterminer (735) si un pourcentage du nombre de réflectances, qui sont inférieures à la valeur seuil, est un pourcentage prédéterminé ou plus par rapport au nombre total de réflectances,
si ce n'est pas le cas, déterminer que seule une zone partielle de la peau est changée en couleur et conseiller (736) à un utilisateur de changer une position de mesure de capteur,
si tel est le cas, déterminer que toute la zone de peau est changée en couleur et changer (737) la condition de fonctionnement de capteur en une seconde condition de fonctionnement.

2. Support de stockage lisible par ordinateur selon la revendication 1, dans lequel une première source lumineuse (111a) de la pluralité de sources lumineuses (111a, 111b, 111c, 111d) a une longueur d'onde centrale dans une première plage allant d'environ 350 nm à environ 450 nm ou dans une seconde plage allant d'environ 500 nm à environ 600 nm.

3. Support de stockage lisible par ordinateur selon la revendication 1 ou 2,
dans lequel la quantité de lumière initiale est détectée à partir d'un réflecteur standard utilisant la première source lumineuse (111a) sur la base de la première condition de fonctionnement, et
dans lequel le processeur (120) est en outre configuré pour déterminer, en tant que première réflectance, un résultat obtenu en divisant une première quantité de lumière par la quantité de lumière initiale.

4. Support de stockage lisible par ordinateur selon l'une des revendications 1 à 3, dans lequel le processeur (120) est en outre configuré pour :
obtenir (640) un spectre cutané sur la base de la pluralité de quantités de lumière,
déterminer une absorbance sur la base du spectre cutané obtenu, et
estimer (660) une concentration d'un composant analyte sur la base de l'absorbance déterminée.

5. Support de stockage lisible par ordinateur selon l'une des revendications 1 à 4, dans lequel le processeur (120) est en outre configuré pour, sur la base d'une condition prédéterminée qui est satisfaite, déterminer la condition de fonctionnement du capteur (110), et/ou
dans lequel la condition de fonctionnement du capteur (110) comprend un courant d'entraînement pour entraîner la pluralité de sources de lumière (111a, 111b, 111c, 111d).

6. Support de stockage lisible par ordinateur selon l'une des revendications 1 à 5, dans lequel le composant analyte comprend au moins l'un parmi un caroténoïde cutané, caroténoïde sanguin, glucose, urée, lactate, triglycéride, protéine totale, cholestérol et éthanol.

7. Procédé d'estimation d'une concentration d'un composant, le procédé comprenant les étapes consistant à :
établir (731) une condition de fonctionnement d'un capteur (110) comportant une pluralité de sources lumineuses (111a, 111b, 111c, 111d) et une pluralité de détecteurs (112) à une première condition de fonctionnement ;
mesurer (732) une pluralité de quantités de lumière cutanées en entraînant une première source lumineuse (111a) sur la base de la première condition de fonctionnement et en utilisant la pluralité de détecteurs (112) ;
calculer (733) des réflectances cutanées pour chacun des détecteurs (112) en utilisant les quantités de lumière cutanées mesurées par les détecteurs respectifs (112) et une quantité de lumière initiale ;
comparer les réflectances cutanées pour chacun des détecteurs (112) à une valeur seuil et déterminer (734) s'il existe une réflectance inférieure à la valeur seuil ;
si toutes les réflectances sont supérieures ou égales à la valeur seuil, maintenir la première condition de fonctionnement courante ;
s'il existe une réflectance inférieure à la valeur seuil, déterminer (735) si un pourcentage du nombre de réflectances, qui sont inférieures à la valeur seuil, est un pourcentage prédéterminé ou plus par rapport au nombre total de réflectances ;
si ce n'est pas le cas, déterminer que seule une zone partielle de la peau est changée en couleur et conseiller (736) à un utilisateur de changer une position de mesure de capteur,
si tel est le cas, déterminer que toute la zone de peau est changée en couleur et
changer (737) la condition de fonctionnement de capteur en une seconde condition de fonctionnement.

8. Procédé selon la revendication 7, dans lequel la quantité de lumière initiale est détectée à partir d'un réflecteur standard à l'aide d'une première source lumineuse (111a) sur la base de la première condition de fonctionnement.

9. Procédé selon la revendication 7 ou 8, comprenant en outre l'estimation d'une concentration d'un composant d'analyte, comportant :
l'obtention (620) d'un spectre cutané sur la base de la pluralité de quantités de lumière ;
la détermination d'une absorbance à l'aide du spectre cutané obtenu ; et
l'estimation (660) de la concentration du composant d'analyte sur la base de l'absorbance déterminée.

10. Dispositif à porter sur soi (800) comprenant :
un corps principal porté sur un corps d'un utilisateur ;
un capteur (110, 810) comprenant :
une pluralité de sources lumineuses (111a, 111b, 111c, 111d) ayant différentes longueurs d'onde centrales, et
une pluralité de détecteurs (112) configurés pour détecter de la lumière provenant de la peau du corps de l'utilisateur ; et
un processeur (120) configuré pour :
établir (731) une condition de fonctionnement du capteur (110, 810) à une première condition de fonctionnement,
mesurer (732) une pluralité de quantités de lumière cutanées en entraînant une première source lumineuse (111a) sur la base de la première condition de fonctionnement du capteur (110, 810) et en utilisant la pluralité de détecteurs (112),
calculer (733) des réflectances cutanées pour chacun des détecteurs (112) en utilisant les quantités de lumière cutanées mesurées par les détecteurs respectifs (112) et une quantité de lumière initiale,
comparer (734) les réflectances cutanées pour chacun des détecteurs (112) à une valeur seuil et déterminer s'il existe une réflectance inférieure à la valeur seuil,
si toutes les réflectances sont supérieures ou égales à la valeur seuil, maintenir la première condition de fonctionnement courante,
s'il existe une réflectance inférieure à la valeur seuil, déterminer (735) si un pourcentage du nombre de réflectances, qui sont inférieures à la valeur seuil, est un pourcentage prédéterminé ou plus par rapport au nombre total de réflectances,
si ce n'est pas le cas, déterminer que seule une zone partielle de la peau est changée en couleur et conseiller (736) à un utilisateur de changer une position de mesure de capteur,
si tel est le cas, déterminer que toute la zone de peau est changée en couleur et changer (737) la condition de fonctionnement de capteur en une seconde condition de fonctionnement.

11. Dispositif à porter sur soi (800) selon la revendication 10, dans lequel la première condition de fonctionnement est établie à l'avance sur la base d'au moins l'une parmi :
une période de temps pendant laquelle le corps principal n'est pas porté sur le corps de l'utilisateur avant l'estimation d'un composant antioxydant,
une période de temps au cours d'une journée pendant laquelle le corps principal n'est pas porté sur le corps de l'utilisateur, et
une saison.
